# EUROPEAN PATENT SPECIFICATION

(11) **EP 2 279 266 B1**
(45) Date of publication and mention of the grant of the patent: **11.10.2017**
(21) Application number: 09725711.7
(22) Date of filing: 25.03.2009
(51) Int. Cl.: C12Q 1/68

(54) **A METHOD FOR DETECTING IGF1R ABERRATIONS IN CIRCULATING TUMOR CELLS USING FISH**
VERFAHREN ZUM NACHWEIS VON IGF1R-ABERRATIONEN IN ZIRKULIERENDEN TUMORZELLEN UNTER VERWENDUNG VON FISH
PROCÉDÉ DE DÉTECTION D'ABERRATIONS DE IGF1R DANS CELLULES TUMORALES CIRCULANTES PAR FISH

(30) Priority: 25.03.2008 US 39162 P
(43) Date of publication of application: 02.02.2011
(73) Proprietor: Janssen Diagnostics, LLC, Raritan, NJ 08869 (US)
(72) Inventor: FOULK, Brad, Chalfont PA 18914-2262 (US); TERSTAPPEN, Leon, W.M.M., NL-1095 DW Amsterdam (NL)
(74) Representative: Goodfellow, Hugh Robin
(86) International application number: PCT/US2009/038226
(87) International publication number: WO 2009/120767

(56) References cited:
- WO-A1-2007/053142
- WO-A1-2007/112746
- WO-A2-2007/053245
- US-A1- 2005 059 021
- US-A1- 2006 234 239
- US-B1- 6 365 362
- US-B1- 6 475 720
- SMIRNOV D A ET AL: "Global Gene Expression Profiling of Circulating Endothelial Cells in Patients with Metastatic Carcinomas", CANCER RESEARCH, AMERICAN ASSOCIATION FOR CANCER REREARCH, US, vol. 66, no. 6, 15 March 2006 (2006-03-15) , pages 2918-2922, XP003009584, ISSN: 0008-5472, DOI: DOI:10.1158/0008-5472.CAN-05-4003
- SMIRNOV D A ET AL: "GLOBAL GENE EXPRESSION PROFILING OF CIRCULATING TUMOR CELLS", CANCER RESEARCH, AMERICAN ASSOCIATION FOR CANCER REREARCH, US, vol. 65, no. 12, 15 June 2005 (2005-06-15) , pages 4993-4997, XP003001873, ISSN: 0008-5472, DOI: DOI:10.1158/0008-5472.CAN-04-4330
- GILES HELLAWELL ET AL: "Expression of the type 1 insulin-like growth factor receptor is up-regulated in primary prostate cancer and commonly persists in metastatic disease.", CANCER RESEARCH, vol. 62, no. 10, 1 May 2002 (2002-05-01), pages 2942-50, XP55001216, ISSN: 0008-5472
- BALSARA ET AL.: 'Comparative genomic hybridiation analysis detects frequent, often high-level, overrepresentation of DNA sequences at 3q, 5p, 7p, and 8q in human non-small cell lung carcinomas' CANCER RESEARCH vol. 57, 1997, pages 2116 - 2120, XP008145062
- JOSÉ ADÉLAÏDE ET AL: "Integrated profiling of basal and luminal breast cancers", CANCER RESEARCH, AMERICAN ASSOCIATION FOR CANCER RESEARCH, US, vol. 67, no. 24, 15 December 2007 (2007-12-15), pages 11565-11575, XP002668606, ISSN: 0008-5472, DOI: 10.1158/0008-5472.CAN-07-2536

## Description

### BACKGROUND OF THE INVENTION

### Field of the Invention

The invention relates generally to the fields of oncology and diagnostic imaging. More specifically, the invention relates to methods in the detection of cancer and for assessing treatment regimens in patients.

### • Background Art

Despite efforts to improve treatment and management of cancer patients, survival in cancer patients has not improved over the past two decades for many cancer types. Accordingly, most cancer patients are not killed by their primary tumor, but they succumb instead to metastases: multiple widespread tumor colonies established by malignant cells that detach themselves from the original tumor and travel through the body, often to distant sites. The most successful therapeutic strategy in cancer is early detection and surgical removal of the tumor while still organ confined. Early detection of cancer has proven feasible for some cancers, particularly where appropriate diagnostic tests exist such as PAP smears in cervical cancer, mammography in breast cancer, and serum prostate specific antigen (PSA) in prostate cancer. However, many cancers detected at early stages have established micrometastases prior to surgical resection. Thus, early and accurate determination of the cancer's malignant potential is important for selection of proper therapy.

Optimal therapy will be based on a combination of diagnostic and prognostic information. An accurate and reproducible diagnostic test is needed to provide specific information regarding the metastatic nature of a particular cancer, together with a prognostic assessment that will provide specific information regarding survival.

A properly designed prognostic test will give physicians information about risk and likelihood of survival, which in turn gives the patient the benefit of not having to endure unnecessary treatment. Patient morale would also be boosted from the knowledge that a selected therapy will be effective based on a prognostic test. The cost savings associated with such a test could be significant as the physician would be provided with a rationale for replacing ineffective therapies. A properly developed diagnostic and prognostic data bank in the treatment and detection of metastatic cancer focusing on survival obviously would provide an enormous benefit to medicine (US 6,063,586).

If a primary tumor is detected early enough, it can often be eliminated by surgery, radiation, or chemotherapy or some combination of those treatments. Unfortunately, the metastatic colonies are difficult to detect and eliminate and it is often impossible to treat all of them successfully. Therefore from a clinical point of view, metastasis can be considered the conclusive event in the natural progression of cancer. Moreover, the ability to metastasize is a property that uniquely characterizes a malignant tumor.

Detection of intact tumor cells in blood provides a direct link to recurrent metastatic disease in cancer patients who have undergone resection of their primary tumor. Unfortunately, the same spreading of malignant cells continues to be missed by conventional tumor staging procedures. Recent studies have shown that the presence of a single carcinoma cell in the bone marrow of cancer patients is an independent prognostic factor for metastatic relapse (Diel IJ, Kaufman M, Goerner R, Costa SD, Kaul S, Bastert G. Detection of tumor cells in bone marrow of patients with primary breast cancer: a prognostic factor for distant metastasis. J Clin Oncol, 10:1534-1539, 1992). But these invasive techniques are deemed undesirable or unacceptable for routine or multiple clinical assays compared to detection of disseminated epithelial tumor cells in blood.

Methods for the characterization of not only tumor cells, but also rare cells, or other biological entities from biological samples have been previously described (US 6,365,362). This two stage method requires efficient enrichment to ensure acquisition of target cells while eliminating a substantial amount of debris and other interfering substances prior to analysis, allowing for cellular examination by imaging techniques. The method combines elements of immunomagnetic enrichment with multi-parameter flow cytometry, microscopy and immunocytochemical analysis in a uniquely automated way. The combination method is used to enrich and enumerate epithelial cells in blood samples, thus providing a tool for measuring cancer.

The two stage method has applications in cancer prognosis and survival for patients with metastatic cancer (WO 04076643). Based on the presence of morphologically intact circulating cancer cells in blood, this method is able to correlate the presence of circulating cancer cells of metastatic breast cancer patients with time to disease progression and survival. More specifically, the presence of five (5) or more circulating tumor cells per 7.5 milliliters provides a predictive value at the first follow-up, thus providing an early prognostic indicator of patient survival.

The specificity of the assay described above increases with the number of cells detected and is not sufficient in cases were only few (generally less than 5 circulating tumor cells) are detected. One solution to this problem is to provide detailed genetic information about suspected cancer cells. Accordingly, a method that would incorporate enrichment of a blood sample with multi-parametric image cytometry and multi-parametric genetic analysis on an individual suspect cancer cell would provide a complete profile and confirmatory mechanism to significantly improve current procedures for patient screening, assessing recurrence of disease, or overall survival.

Fluorescent in situ hybridization (FISH) has been described as a single mode of analysis in rare cell detection after enrichment as described in WO 00/60119; Meng et al. PNAS 101 (25): 9393-9398 (2004); Fehm et al. Clin Can Res 8: 2073-2084 (2002). After epithelial cell enrichment, captured cells are screened by known hybridization methods and imaged on a microscope slide. Because of inherent technical variations and a lack of satisfactory confirmation of the genetic information, the hybridization pattern alone does not provide a level of clinical confidence that would be necessary for sensitive analysis, as in assessing samples with less than 5 target cells. Further, this method for FISH analysis is difficult to automate.

Coupling hybridization-based methods with immunocytochemistry in the analysis of individual cells has been previously described (US 6,524,798). Simultaneous phenotypic and genotypic assessment of individual cells requires that the phenotypic characteristics remain stable after in situ hybridization preparatory steps and are limited in the choice of detectable labels. Typically, conventional in situ hybridization assays require the following steps: (1) denaturation with heat or alkali; (2) an optional step to reduce nonspecific binding; (3) hybridization of one or more nucleic acid probes to the target nucleic acid sequence; (4) removal of nucleic acid fragments not bound; and (5) detection of the hybridized probes. The reagents used to complete one or more of these steps (i.e. methanol wash) will alter antigen recognition in subsequent immunocytochemistry, cause small shifts in the position of target cells or completely removes the target cells, which introduces the possibility of mischaracterization of suspect cells.

The ability to analyze rare circulating cells by combining phenotypic and genotypic multiparametic analysis of an individually isolated target cell would provide confirmation to the clinician of any quantitative information acquired. This is especially relevant when disease states are assessed using extremely small (1, 2, 3, or 4) numbers of circulating tumor cells (CTC's), providing a confirmation for early disease detection.

Probe sets and methods for multi-parametric FISH analysis has been described in lung cancer (US 20030087248). A 3 probe combination resulting in 95% sensitivity for detecting bladder cancer in patients has also been described, see US 6,376,188; US 6,174,681. These methods lack the specificity and sensitivity for assessing small numbers of target cells, and thus a confirmatory assessment for early detection of disease state. They also do not provide a means for convenient automation.

A recently described probe eliminates the repetitive sequences from DNA to provide a repeat-free sequence. The repeat-free probes function as hybridization probes without the use of blocking DNA or the need to block undesired DNA sequences (WO07/053245).

High levels of IGF-1 expression have been associated with an increase risk of cancers such as lung, breast, prostate and colorectal, compared to individuals with lower IGF-1 levels. Further, there is considerable evidence for a role for IGF-1 and/or IGF-1R in the maintenance of tumor cells *in vitro* and *in vivo.* IGF-1R levels are elevated in tumors of lung (Kaiser et al., J. Cancer Res. Clin. Oncol. 119: 665-668, 1993; Moody et al., Life Sciences 52: 1161-1173, 1993; Macauley et al., Cancer Res., 50: 2511-2517, 1990), breast (Pollak et al., Cancer Lett. 38: 223-230, 1987; Foekens et al., Cancer Res. 49: 7002-7009, 1989; Arteaqa et al., J. Clin. Invest. 84: 1418-1423, 1989), prostate and colon (Remaole-Bennet et al., J. Clin. Endocrinol. Metab. 75: 609-616, 1992; Guo et al., Gastroenterol. 102: 1101-1108, 1992). Deregulated expression of IGF-1 in prostate epithelium leads to neoplasia in transgenic mice (DiGiovanni et al., Proc. Nat'l. Acad. Sci. USA 97: 3455-3460, 2000). In addition, IGF-1 appears to be an autocrine stimulator of human gliomas (Sandberg-Nordqvist et al., Cancer Res. 53 (11): 2475-78, 1993), while IGF-1 has been shown to stimulate the growth of fibrosarcomas that overexpress IGF-1R (Butler et al., Cancer Res. 58: 3021-3027, 1998). For a review of the role IGF-1/IGF-1R interaction plays in the growth of a variety of human tumors, see Macaulay, Br. J. Cancer, 65: 311-20, 1992.

Using antisense expression vectors or antisense oligonucleotides to the IGF-1R RNA, it has been shown that interference with IGF-1R leads to inhibition of IGF-1-mediated cell growth (see, e.g., Wraight et al., Nat. Biotech. 18: 521-526, 2000). Growth can also be inhibited using peptide analogues of IGF-1 (Pietrzkowski et al., Cell Growth & Diff. 3: 199-205, 1992; Pietrzkowski et al., Mol. Cell. Biol. 12: 3883-3889, 1992), or a vector expressing an antisense RNA to the IGF-1 RNA (Trojan et al., Science 259: 94-97, 1992). In addition, antibodies to IGF-1R (Arteaga et al., Breast Canc. Res. Treatm. 22: 101-106, 1992; and Kalebic et al., Cancer Res. 54: 5531-34, 1994), and dominant negative mutants of IGF-1R (Prager et al., Proc. Nat'l Acad. Sci. USA 91: 2181-85, 1994; Li et al., J. Biol. Chem. 269: 32558-2564, 1994; Jiang et al., Oncogene 18: 6071-6077, 1999), can reverse the transformed phenotype, inhibit tumorigenesis, and induce loss of the metastatic phenotype.

IGF-1 is also important in the regulation of apoptosis. Apoptosis, which is programmed cell death, is involved in a wide variety of developmental processes, including immune and nervous system maturation. In addition to its role in development, apoptosis also has been implicated as an important cellular safeguard against tumorigenesis (Williams, Cell 65: 1097-1098, 1991; Lane, Nature 362: 786-787, 1993). Suppression of the apoptotic program may contribute to the development and progression of malignancies.

Some studies suggest that expression levels of IGF-1R correlate with clinical outcome. In tumor models, IGF-1R modulates cell proliferation, survival and metastasis and induces resistance to targeted therapies. Inhibition of IGF-1R significantly increases the activity of cytotoxic agents (Cohen, B. e al., Clin. Cancer Res. 11 (5): 2063-73). Inhibition of IGF-1R signaling thus appears to be a promising strategy for the development of novel cancer therapies.

The detection of IGF-1R expression on the surface of CTC's has been associated with predicting the efficacy of IGF-1R antagonist therapy in cancer patients (WO07/141626). However, this method lacks the ability to directly examine the chromosomal arrangement on an individual tumor cell. Analysis of the IGF-1R gene would provide information on the apoptotic or proliferative state of individual tumor cells that express IGF-1R, thus resulting in a more specific assessment of tumorigenesis.

Further, a method that would be able to assess both the presence of IGF-1R and any chromosomal aberrations, coupled with the ability to detect surface expression of IGF-1R, would result in a more direct index of aneuploidy in circulating tumor cells expressing IGF-1R, This, in turn, allows for a more specific and sensitive diagnosis and assessment of disease progression.

WO 2007/053245 describes methods and compositions to obtain unique sequence DNA probes, and both the preservation and genetic analysis of cells that have been identified after immunomagnetic selection and fluorescent labeling.

Adélaïde, J. et al., (2007), Cancer Research, American Association for Cancer research, US, describes the analysis of genome copy number and gene expression in human breast cancer.

### SUMMARY OF THE INVENTION

The present invention provides a direct method for assessing the chromosomal arrangement in IGF-1R circulating tumor cells, wherein the method assesses IGF-1R gene aberrations in circulating tumor cells from a blood sample obtained from a patient, said sample comprising a mixed cell population suspected of containing circulating tumor cells. A more direct analysis method will aid clinicians in predicting the benefit of IGF-1R therapy in cancer patients, and providing for a more specific diagnosis in these patients. The method comprises: a) isolating a subpopulation of epithelial cells by immunomagnetic enrichment; and b) identifying suspect circulating tumor cells; and c) hybridizing said suspect circulating tumor cells with an IGF-1R repeat-free probe configuration capable of detecting chromosomal aberrations which consists of clone sequences containing at least a portion of the IGF-1R locus and a repeat-free probe lacking cross-hybridization specific for the q1 region of chromosome 15.

The present invention provides a kit for determining IGF-1R gene aberrations in circulating tumor cells from a patient sample comprising: a) an IGF-1R repeat-free probe configuration capable of detecting chromosomal aberrations which consists of clone sequences containing at least a portion of the IGF-1R locus; and b) a repeat-free probe lacking cross-hybridization specific for the q1 region of chromosome 15; and c) labeling moieties linked to said (a) and (b).

The present invention further provides a kit for screening patient samples for the presence of circulating tumor cells expressing IGF-1R comprising; a) coated magnetic nanoparticles comprising a magnetic core material, a protein base coating material, and an antibody that binds specifically to tumor cells of epithelial cell origin, the antibody being coupled directly or indirectly to said base coating material; b) a cell dye for excluding sample components other than the tumor cells for analysis; c) a satellite enumeration probe for chromosome enumeration and aneuploidy detection; and d) a repeat-free probe capable of defining the IGF-1R gene locus.

### BRIEF DESCRIPTION OF THE DRAWINGS

**Figure 1****:** Schematic representation depicting the generation of repeat depleted DNA probes from BAC starting DNA. A fragmented whole genome amplification library is denatured and allowed to re-anneal in the presence of excess Cot DNA. DSN digestion of the double strand DNA results in a mixture of single strand unique sequence, available as a template for probe production.
**Figure 2****:** Diagram showing the location of two clones used to identify the IGF-1R gene. The IGF-1R probe was designed by selecting BAC clones containing sequences surrounding the IGF-1R locus.
**Figure 3****:** Mapping of the repeat-free IGF-1R DNA clones is depicted. The IGF-1R clones are hybridized to normal human metaphase chromosomes (RED) along with chromosome 17 alpha satellite control probe (GREEN).
**Figure 4****:** Image of the alpha satellite probe for chromosome 15 (SE-15) is shown at the IGF-1R locus. Repeat-free IGF1R clones (RED) were pooled together along with the SE-15 probe (GREEN) and hybridized to normal metaphase chromosome spreads.
**Figure 5****:** Hybridization of the IGF-1R probe configuration on LNCAP cells (right) and BT474 cells (left).
**Figure 6****:** Diagram showing the relative location of probes used in combination. The two cones were repeat-free, fluorescently labeled (RED) probes mapped using the IGF-1R probe (BLUE) as a reference.
**Figure 7****:** The spectra of the fluorochromes used in the assay are shown. ULS DY415 was used for IGF-1R.
**Figure 8****:** Images of seven cell lines assessed with the IGF-1R probe, demonstrating that the probe is capable of detecting chromosomal aberrations.
**Figure 9****:** The ability to score images acquired from the CellTracks instrument is shown in the figure. Images from the CellTracks instrument are compared with a standard fluorescence microscope.
**Figure 10****:** Image of a screenshot form FISH software used in scoring FISH results. The top row shows the original images from the CTC scan, CK+;DAPI+/CD45- and a CTC. The lower row shows a CTC with an aberrant number of IGF-1R and Chromosome 15 probes.

### DETAILED DESCRIPTION OF THE INVENTION

The present invention incorporates the isolation and identification of circulating tumor cells using immunomagnetic enrichment and image analysis coupled to fluorescent *In Situ* hybridization (FISH) in order to detect IGF-1R in tumor cells having aberrant genetic profiles.

To this end, the CellTracks System utilizes immunomagnetic selection and separation to highly enrich and concentrate any epithelial cells present in whole blood samples. The captured cells are detectably labeled with a leukocyte specific marker and with one or more tumor cell specific fluorescent monoclonal antibodies to allow identification and enumeration of the captured CTC's as well as unequivocal instrumental or visual differentiation from contaminating non-target cells. At an extraordinary sensitivity of 1 or 2 epithelial cells per 7.5- 30 ml of blood, this assay allows tumor cell detection even in the early stages of low tumor mass. The embodiment of the present invention is not limited to the CellTracks System, but includes any isolation and imaging protocol of comparable sensitivity and specificity.

DNA contains unique as well as repetitive sequences. The repetitive sequences occur throughout the chromosomes and have the potential to interfere with hybridization reactions, such as with *in situ* hybridization, targeted toward specific regions or unique sequences outside these repetitive sequences. To identify the presence, amount and location of specific sequences on chromosomes, genes or DNA sequences it is important that the hybridization probes hybridize only at the location of interest. The presence of repetitive sequences in the hybridization probe mixture reduces the specificity of the binding, requiring methods to either remove the repetitive sequences from the probes or prevent the probes from hybridizing to the repetitive sequences on the target. For example, Cot-1 DNA is often added during hybridization to prevent binding of the probes to the repetitive sequences (US 5,447,841 and US 6,596,479).

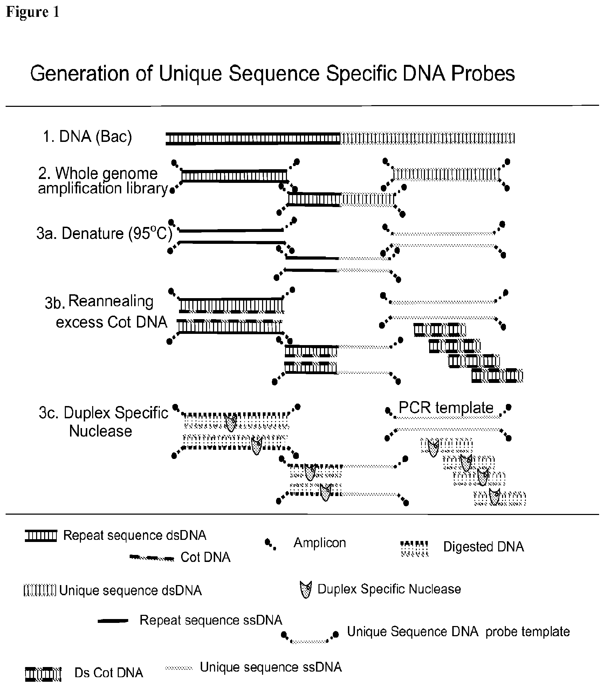

A general diagram for the generation of repeat-depleted DNA probes is depicted in Figure 1. Duplex specific nucleases (DSN) which preferentially cleave deoxyribonucleic acid molecules (US 2005/0164216 and US 6,541,204) preferentially cleave nucleic acid duplex polynucleotides as compared to single strand DNA provides a means for removing non-target double stranded DNA from the sample mixture. The ability of these nucleases to preferentially digest the duplex form of polynucleotides provides potential use in manufacturing unique target specific probe, eliminating the interfering affect of blocking DNA, and providing a means for their rapid, efficient and cost effective production.

Starting DNA is typically in the form of one or more DNA sequences which contain a multiplicity of DNA segments. The initial source of individual starting material in the production of the probe composition has been described in the production of direct-labeled probes (US 6,569,626). Optimally the source of the starting polynucleotide is purified from tissue and fragmented into 150 kb to 200 kb segments, using any known technique such as enzyme treatment (restriction enzymes), polymerase, limited DNase I digestion, limited mung bean nuclease digestion, sonication, shearing of DNA, and the like. Some of these segmental fragments will be complementary to at least a portion of one or more DNA segments in the particular unique target sequence.

The individual DNA segments are propagated by commonly known methods, such as cloning into a plasmid construct and then transfecting into bacteria.

After propagating the cloned fragments, individual colonies representing isolated fragments are identified as containing at least a portion of the sequence of interest. Identification is accomplished by known techniques such as hybridization, PCR, or searching established databases of commercially available libraries. Each chosen colony is grown to obtain an isolated plasmid construct having a unique fragment, at least partially complementary to a segment of the target sequence on the chromosome (i.e. BAC clones).

Once the cloned fragments of interest are propagated and isolated, they are depleted of their repetitive polynucleotide sequences. Using whole gene amplification (WGA), the fragments are amplified as 200 to 500bp segments from the isolated plasmid constructs. Cot-1 DNA is combined with the WGA library pool after amplification by first heating to 95°C to denature the double-strand polynucleotide into a single strand state and then cooling to 65°C to allow selective re-annealing of the repeat sequences. Duplex specific nucleases (DSN) under optimized DSN conditions are then added to preferentially cleave deoxyribonucleic acid molecules containing perfectly matched nucleic acid duplexes while not affecting any remaining single stranded segments. Selectively cleaving the duplex nucleic acids is accomplished by enzymatic digestion of DNA-DNA duplexes and DNA-RNA duplexes. DSN isolated from the Kamchatka crab (US 10/845,366) or shrimp (US 6,541,204) removes the duplex structure. The use of endonuclease-specific nucleases hydrolyzes a phosphodiester bond in the duplex DNA backbone, providing the advantage of not being nucleotide sequence-specific and therefore applicable to most targets of interest. DSN digestion provides for the removal of a substantial amount of the nucleic acid duplex for subsequent amplification of the remaining single-strand polynucleotide. The resulting digestion contains single stranded DNA, corresponding to portions of the unique target sequence on the chromosome, some amount of undigested double-strand DNA, and digested base pairs. Undigested DNA is separated from the digested DNA and the DSN by centrifugation (i.e. spin column chromatography). The mixture is used immediately or stored at 80°C, either before or after amplification of the purified composition for subsequent utilization such as labeling and *in situ* hybridization. After amplification, the resulting target probe sequence is amplified by PCR yielding 90% to 99% pure target probe sequence, and designated repeat-depleted DNA.

The resulting probes are incorporated in the methods embodied in the present invention. Repeat-depleted DNA, as described in the present invention, is useful for in situ hybridization, including FISH as applied to the IGF-1R gene in suspect CTC. The requirement for competitive binding is eliminated using the repeat-depleted probes described in this invention, resulting in increased specificity of the reaction and a reduction in the amount of probes necessary for binding.

### Reanalysis of Immunomagnetically-Labeled Cells

Chromosomal aneuploidy is associated with genetic disorders, particularly cancer. Diagnostic methods are available that provide for the detection of these chromosomal abnormalities particularly with the use of *in situ* hybridization (ISH). The application of ISH and immunocytochemistry (ICC) on tissue or cell samples has been well established, but there is a clear need to establish a diagnostically effective method for the simultaneous analysis of ISH and ICC on a single cell. The present invention provides for the detection of these chromosomal abnormalities on individual cells as they relate to the IGF-1R locus on cancer cells using a cost effective and highly specific means.

One aspect of the present invention involves the further processing of rare cells after enrichment and immunocytochemical (ICC) analysis. For example, circulating rare cells such as epithelial cells are identified as suspect cancer cells (US 6,365,362; US 6,645,731; and US 11/202,875). Suspect cells are identified through specific cellular antigens and nucleic acid labeling. IGF-1R confirmation of these suspect cells are subsequently determined by the expression of specific unique target sequences, defining either a chromosome and/or gene, used to assess chromosomal changes (i.e. aneuploidy) within the identified suspect cell. Accordingly, one embodiment of the present invention includes the combination of ICC staining and subsequent confirmation by ftuorescent *in situ* hybridization (FISH) using a satellite enumeration probe (SE) and a unique sequence probe, capable of binding to a gene or group of genes.

The method provides an increased specificity after immunomagnetic enrichment and fluorescent imaging of circulating tumor cells as provided by the CellTracks® AutoPrep® and CellTracks® Analyzer II Systems (Veridex, LLC) and further described in US 6,365,362. A method permits the designation of 1 or more CTC's as an IGF-1R positive cancer cell regardless of the stage of the disease and thus lowers the threshold for calling a sample positive for CTCs. One embodiment of the present invention is to detect the presence or absence of IGF-1R as a therapeutic target and thus provides a means to make the correct choice of treatment.

Accordingly, an automated and standardized method for blood sample processing provides identification of circulating epithelial cells by ICC. Aspirated plasma from a partitioned blood sample is combined with a ferrofluid reagent conjugated to antibodies specific for a target cell population (i.e. EpCAM positive). These cells are immunomagnetically collected through an externally applied magnetic field, allowing for separation and removal of unlabeled cells.

Once the target cells are separated, they are dispensed into a disposable cartridge for image analysis using an image presentation device (US 6,790,366 and US 6,890,426). The device is designed to exert a magnetic field that orients the labeled cells along the optically transparent surface of the chamber for subsequent ICC imaging.

After ICC imaging, suspect cells are identified using appropriate algorithms. Images of the suspected cells are presented to the user who makes the final decision about the identity of the presented suspect cells. Images of the suspect cells and their relative position along the optically transparent viewing surface of the chamber are recorded and archived for later use.

Since ICC imaging alone lacks the specificity to assess the clinical significance of blood samples with less than 5 CTC's or to provide detailed genetic information about suspected cancer cells, subsequent analysis on individual suspect cells is needed to provide a complete profile and establish confirmation that a selected suspect tumor cell can be used in diagnostic analysis, including screening, assessing recurrence of disease, and overall survival.

FISH requires temperatures above the melting temperature of DNA as well a reagents that are not compatible with the ICC labeling. Most of the ICC and DNA labels do not survive the FISH procedure with any signals lost in processing. Thus, a cell that was identified as being an interesting cell for FISH analysis can not be traced back on its position. Therefore there is a need to have a detection method that once the ICC image is obtained, the cell position along the optically transparent viewing surface is maintained for subsequent genetic analysis (FISH) or other types of analysis in which the ICC labels are lost. This is achieved, in part, by fixing the cells on the optically transparent surface after the ICC image is obtained without a loss of cells or any substantial movement along the surface.

Accordingly after addition of the FISH reagents, the cartridge is placed on a hotplate having the surface with the immobilized cells in contact with the hotplate. Depending on the type of assay the hotplate is programmed with different temperature cycles that run between 2 and 48 hours. After the temperature cycles are completed, the excess FISH reagents are removed from the cartridge. The cartridge is filled with a buffer solution containing a DNA label to visualize the nuclei of immobilized cells. Depending on the DNA label used, the label remains in the cartridge or is washed out of the cartridge after staining.

Next, the cartridge is placed back in the CellTracks® Analyzer II System for a second scan. Because cells present on the upper surface during the first ICC image analysis were immobilized, the same cells are still in the same relative location inside the cartridge. To assess the shift of the cartridge relative to the imaging system (CellTracks® Analyzer II System), the locations of the nuclei in the images of the second scan are compared to the location of the nuclei in the images of the first ICC scan. The shift of these images with respect to each other is determined using convolution algorithms. After this shift has been determined a specific cell of interest, based on its ICC image, can be selected from a list and be relocated on the surface of cartridge after FISH in the second scan. Next fluorescent images of selected FISH probes are acquired.

### Example 1- Development of a Probe Set for an IGF-1R/Chr 15 FISH Assay.

Two types of probes are needed for the assay. One type is a satellite enumeration (SE) probe. These probes bind the satellite (repetitive) sequence near the centromere of the chromsome and is used for chromosome enumeration and aneuploidy detection. The second type of probe is the unique sequence probe. As the name implies these probes bind unique sequences, like genes. Using bioinformatics, unique sequence probes can be designed for any location in the genome. Unique sequece probes usually contain repetitive elements like Alu or Kpn repeats which can cause non-specific binding of the probe. Suppression of non-specific binding is typically done by the incorporation of unlabeled blocking DNA in the hybridization. Blocking DNA has been shown to interfere with hybridization of unique sequence and satellite probes. The present method eliminates this step with the use of repeat-free probes which allow faster, brighter hybridization of the probes without the drawbacks of using of blocking DNA.

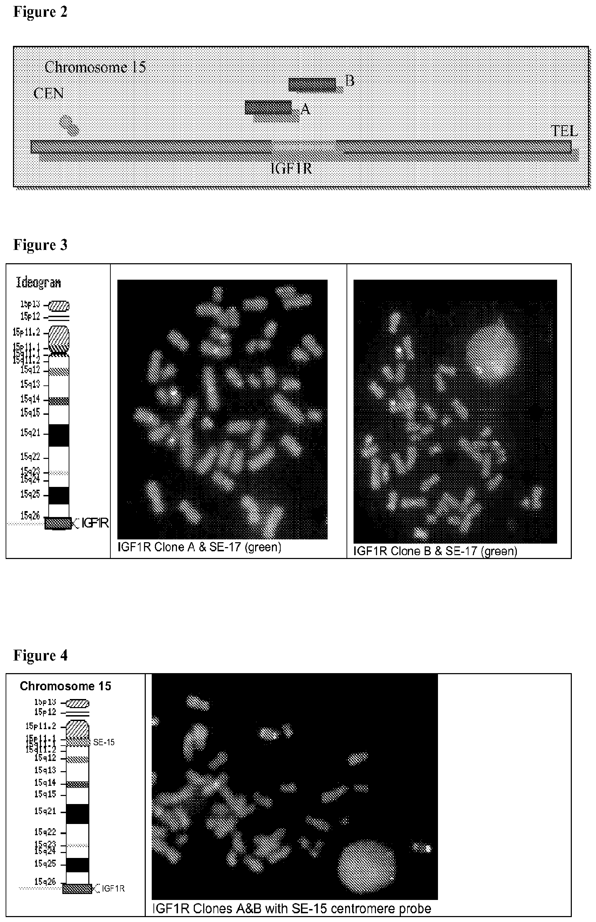

Using bioinformatics, an IGF-1R probe was designed by selecting bacterial artificial chromosome (BAC) clones that contain sequences surrounding the IGF1R locus. The diagram in Figure 2 shows the location of the two clones with respect to the IGF1R gene. BAC DNA was propagated and isolated using the Qiagen Large Construct Kit. Clones were FISH mapped for specificity and then made repeat-free according to the protocol described. From Figure 2, the "A" and "B" in RED represent the two clones surrounding the IGF-1R locus. The GREEN region represents the satellite enumeration probe on chromosome 15 (SE-15).

The IGF1R clones were mapped to confirm their function. Amplified repeat-free DNA for both clones was sonicated and labeled with ULS- PlatinumBright 550 (Kreatech Diagnostics). As shown in Figure 3, IGF1R clones (RED) were hybridized to normal human metaphase chromosomes along with a chromosome 17 alpha satellite control probe (GREEN). Both clones give bright signals on the q-terminus of an areocentric chromosome of the appropriate size. No cross-hybridization of the repeat free clones was seen on other chromosomes.

An alpha satellite probe for chromosome 15 (SE-15) was evaluated for use as a reference probe for the IGF-1R locus. As shown in Figure 4, repeat-free IGF-1R clones A & B (RED) were pooled together along with the SE-15 centromere probe (green) and hybridized to normal metaphase chromosome spreads. The images below clearly demonstrate that the IGF1R and the SE15 probes map to the correct chromosome and give strong signals.

### Example 2- Cell Line Evaluation of the IGF-1R/SE-15 Probe Configuration.

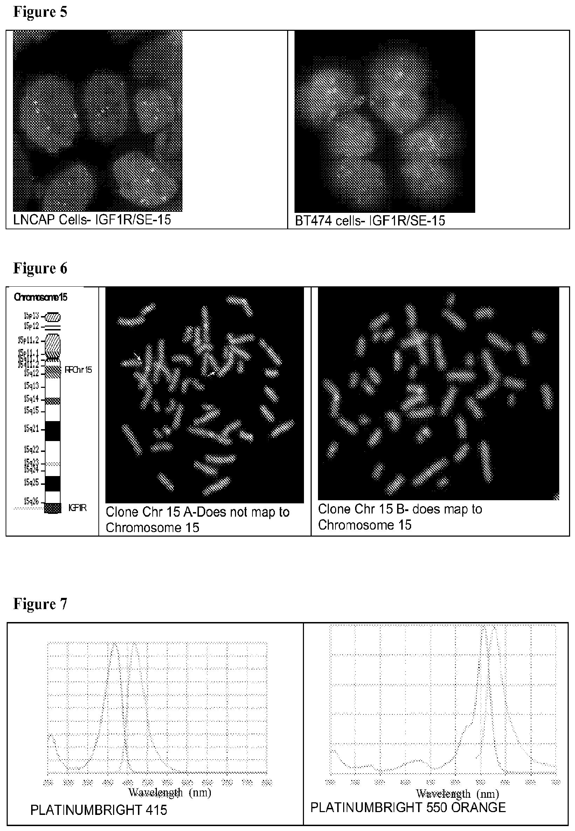

The combination of the alpha satellite probe for chromosome 15 and the IGF-1R clones A & B (IGF-1R/SE-15) were tested for assessing genetic aberrations. The IGF1R/SE-15 probe configuration was used on several cell lines to see if the probe could detect aneupliody or gene amplification/deletion. A549 (lung), BT474 (Breast), PC3 (prostate), LNCAP (prostate), H1299 (lung), and MCF7 (Breast) cell lines were tested with this configuration. The images in Figure 5 show that LNCAP have four copies of SE-15 and four copies of IGF1R, indicating aneupliody with no gene amplification. The remaining five cell lines tested showed cross-hybridization of the SE-15 probe to other chromosomes. BT 474 cells contain 2-3 copies of IGF1R and numerous SE-15 signals indicating cross-hybridization. The other cell lines had varying amounts of cross hybridization of the SE-15 probe. Since the degree of cross hybridization varied among the cell lines and normal donors tested it is likely that this cross hybridization is due to donor to donor variables.

In order to resolve the SE-15 cross-hybridization issue, the IGF-1R/SE-15 probe was reconfigured with a unique sequence probe for a locus elsewhere on Chromosme 15. Since this is a unique sequence probe it should bind to a specific region of Chromosome 15 without the cross hybridization problems that can arise from using a alpha satellite probe like SE-15. Two adjacent clones were selected in the ql region of Chromosome 15, located near the centromere on the same arm of chromosome 15 as IGF1R. The diagram in Figure 6 shows the relative location of probes used in this configuration. The two clones were made according to the repeat-free protocol and fluorescently labeled (RED). The IGF-1R probe (BLUE) was used as reference for mapping. As shown in the image, the clone Chr 15 A mapped to an incorrect chromosome. Clone Chr 15 B mapped correctly to chromosome 15 and was further evaluated as a reference probe for IGF-1R.

Figure 7 shows the spectra for the fluorochomes used as a label for the probes. The ULS-550 dye (Kreatech Diagnostics) was selected for the Chr 15 probe while ULS DY415 was used for IGF-1R.

Optimum concentration for each probe in the configuration was based on the brightest signal with the best signal to noise measurement. Hybridization mixes consisted of 50% formamide, 1xSSC, 10% Dextran sulfate, 10% Kreaboost and varying probe concentrations. White blood cells fixed in CellTracks cartridges were FISHed overnight and a Stringency wash was performed using 24% Formamide and 0.1xSSC. Signal and background intensities were measured by acquiring fluorescent images of 10 cells and dividing the intensity of the brightest pixel (signal) by the average intensity of several random pixels in the nucleus of the cell (noise). The concentrations tested and the associated signal to noise ratios (SNR) are listed in Table I. The optimal concentrations have been determined to be 4ng/µl for both IGF1R and Chr 15.

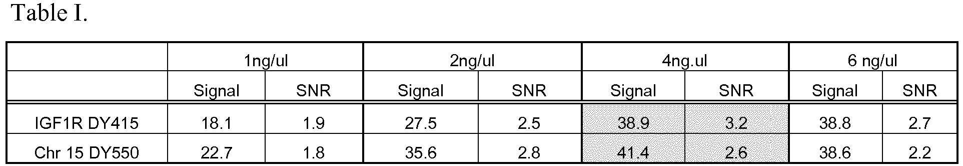

### Example 3- Evaluation of Probe Configuration from Donor Samples.

After obtaining the optimum conditions for the assay which included the use of 4 ng/µl of each probe in the reaction, the probe configuration was evaluated on a total of 350 CD45⁺/CK⁻ cells (leukocytes) from three different donors and analyzed using the CellTracks-FISH System. The number of copies of each target scored and the results are listed in the tables below. FISH signals were able to be scored in greater than 95% of the cells relocated by the CellTracks-FISH System.

Table II shows the results from the three donors. The expected 2 copies ofIGFIR occurred in 87%, 81 %, and 93 % of the WBC examined from three donors. It was expected that ≥75% of WBC should show the expected result of 2 dots per WBC for IGF1R and >85% of the WBC evaluated should be scoreable. The three donors showed the expected 2 copies of Chr 15 in 91%, 97%, and 93% of the WBC examined. For this reason we set a QC specification that ≥80% of WBC should show the expected result of 2 dots and >85% of the WBC evaluated should be scoreable. The data is consistent with data collected in a similar fashion using other selected probes.

**Table II.**

| Donor 887 | Cells evaluated | # Cells 0 copies | # Cells 1 copy | # Cells 2 copies | # Cells 3 copies |
|---|---|---|---|---|---|
| IGF1R | 128 | 0 | 14 (11%) | 111 (87%) | 3 (2%) |
| Chr 15 | 129 | 0 | 11 (9%) | 116 (91%) | 2 (2%) |

| Donor 888 | Cells evaluated | # Cells 0 copies | # Cells 1 copy | # Cells 2 copies | # Cells 3 copies |
|---|---|---|---|---|---|
| IGF1R | 32 | 0 | 6 (19%) | 26 (81%) | 0 |
| Chr 15 | 33 | 0 | 1 (3%) | 31 (97%) | 1 (3%) |

| Donor 889 | Cells evaluated | # Cells 0 copies | # Cells 1 copy | # Cells 2 copies | # Cells 3 copies |
|---|---|---|---|---|---|
| IGF1R | 194 | 0 | 13 (7%) | 180 (93%) | 1 (1%) |
| Chr 15 | 194 | 0 | 13 (7%) | 180 (93%) | 1 (1%) |

### Example 4- Detection of Chromosomal Aberrations.

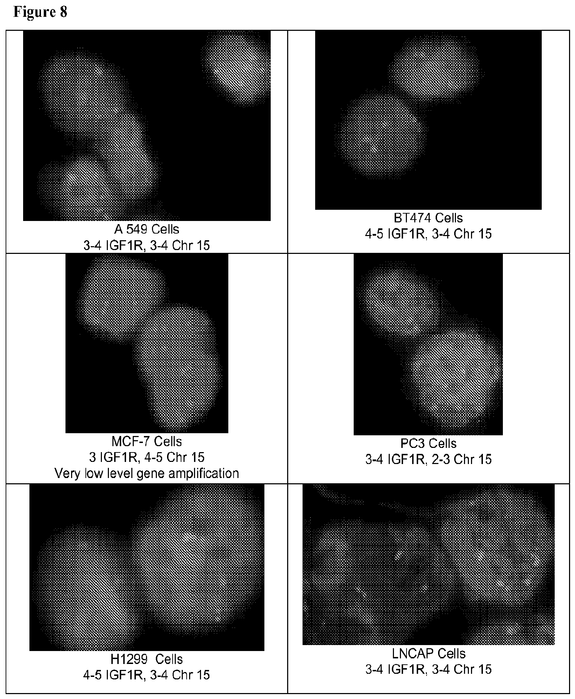

Seven cell lines were assayed with the optimized IGF-1R probe to demonstrate that the probe is capable of detecting chromosomal aberrations. As shown in Figure 8, the microscope images depict IGF-1R signals (BLUE) and the Chr 15 signals (RED) along with the typical counts seen when viewing numerous cells. All cell lines examined contained extra copies of both IGF-1R and Chr 15 probe. Most of the gains in copy number were balanced gains of both probes indicating aneupliody rather that gene amplification. The exception to this is the MCF7 cell line which consistently had 1-2 extra copies of IGF-1R with respect to the Chr 15 probe indicating a low level gene amplification. No cell line tested showed high level IGF-1R gene amplification.

### Example 5- Comparision Between the Automated CellTracks Instrument and a Standard Fluorescence Microscope.

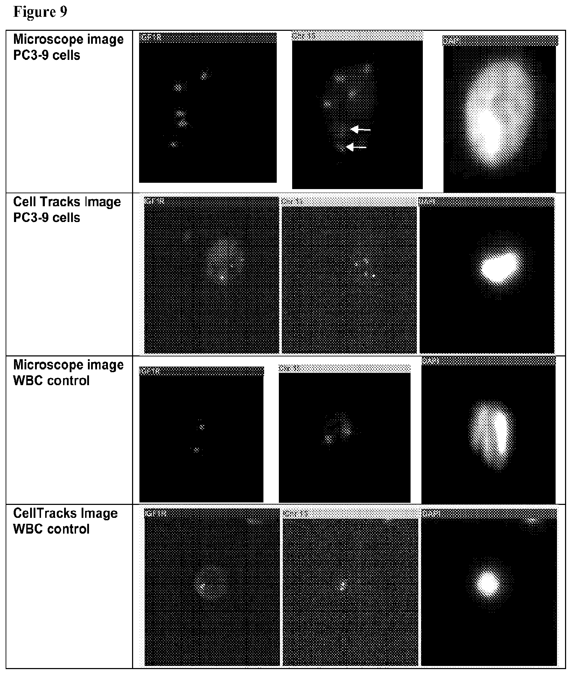

To verify that the CellTracks instrument is capable of acquiring images useful in automated analysis, images of cells from the same cartridge were acquired with both the CellTracks-FISH instrument and a standard fluorescence microscope. Figure 9 shows images of a representative PC3-9 cells and white blood cells control. The figure demonstrates that images from the CellTracks instrument are at least as good than images from the microscope. The Chr 15 image of the PC3-9 cell taken with the microscope shows two dots that are a little out of focus (white arrows). Conversely, the CellTracks instrument takes images in several focal planes and creates a composite image where all dots are in focus, allowing for easier interpretation. Typical PC3-9 cells showed 4-6 copies of Chr 15 and 3-5 copies of IGF-1R.

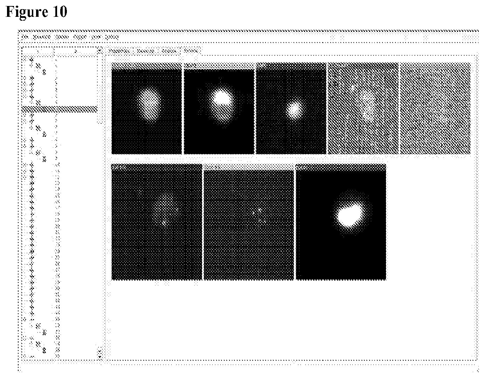

The CellTracks-FISH System is an ideal platform for automated IGF-1R analysis of a fluid sample. Figure 10 shows a typical screenshot image of selected cells after enrichment and fixation in the cartridge. The top row of images shows original images from the CTC scan showing that this cell is CK⁺DAPI⁺/CD45⁻ and, therefore, a suspect CTC. The lower row of images are acquired by the FISH instrument showing that the CTC has an aberrant number of IGF1R and Chr 15 probes. The FISH software scores the results and provides the information for diagnostic interpretation. This demonstrates that CTC from a patient sample can be enriched from blood, fixed in the cartridge and analyzed using FISH techniques in the CellTracks System.

## Claims

1. A method for assessing IGF-1R gene aberrations in circulating tumor cells from a blood sample obtained from a patient, said sample comprising a mixed cell population suspected of containing circulating tumor cells, comprising:
a. isolating a subpopulation of epithelial cells by immunomagnetic enrichment;
b. identifying suspect circulating tumor cells; and
c. hybridizing said suspect circulating tumor cells with an IGF-1R repeat-free probe configuration capable of detecting chromosomal aberrations which consists of clone sequences containing at least a portion of the IGF-1R locus and a repeat-free probe lacking cross-hybridization specific for the q1 region of chromosome 15.

2. The method of Claim 1 wherein said immunomagnetic enrichment comprises:
a. mixing said sample with colloidal immunomagnetic particles coupled to a ligand which binds specifically to suspect circulating tumor cells, to the substantial exclusion of other populations; and
b. subjecting the sample-immunomagnetic particle mixture to a high gradient magnetic field to produce a separated cell fraction enriched in immunomagnetic particle-bound tumor cells.

3. The method of Claim 1 wherein said phenotypic profile is determined from a method selected from a group consisting of multiparameter flow cytometry, immunofluorescent microscopy, laser scanning cytometry, bright field base image analysis, capillary volumetry, spectral imaging analysis, manual cell analysis, automated cell analysis and combinations thereof.

4. The method of Claim 3 wherein said phenotypic profile is determined from automated immunofluorescent cell analysis.

5. The method of Claim 4 wherein said clone sequence is selected from bacterial artificial chromosome clones.

6. The method of Claim 1 wherein said hybridizing suspect target cell provides diagnostic, prognostic, or therapeutic information of said patient.

7. A kit for determining IGF-1R gene aberrations in circulating tumor cells from a patient sample comprising:
a. an IGF-1R repeat-free probe configuration capable of detecting chromosomal aberrations which consists of clone sequences containing at least a portion of the IGF-1R locus;
b. a repeat-free probe lacking cross-hybridization specific for the q1 region of chromosome 15; and
c. labeling moieties linked to said (a) and (b).

8. The kit of Claim 7 wherein said polynucleotide probe sequence is depleted of said repetitive sequences by digestion with duplex-specific nuclease.

9. The kit of Claim 7 wherein said labeling moiety is a fluorophore linked by a platinum-based coordinative bond.

## Patentansprüche

1. Verfahren zur Untersuchung von IGF-1R-Gen-Aberrationen in zirkulierenden Tumorzellen aus einer von einem Patienten erhaltenen Blutprobe, wobei die Probe eine gemischte Zellpopulation umfasst, von der vermutet wird, dass sie zirkulierende Tumorzellen enthält, umfassend:
a. Isolieren einer Subpopulation von Epithelzellen durch immunmagnetische Anreicherung,
b. Identifizieren vermuteter zirkulierender Tumorzellen und
c. Hybridisieren der vermuteten zirkulierenden Tumorzellen mit einer Repeat-freien IGF-1R-Sondenkonfiguration, die in der Lage ist, Chromosomenaberrationen zu detektieren und die aus Klonsequenzen besteht, die mindestens einen Abschnitt des IGF-1R-Locus enthalten, und mit einer Repeat-freien Sonde ohne Kreuzhybridisierung, die für die ql-Region von Chromosom 15 spezifisch ist.

2. Verfahren nach Anspruch 1, wobei die immunmagnetische Anreicherung Folgendes umfasst:
a. Mischen der Probe mit kolloidalen immunmagnetischen Partikeln, die an einen Liganden gekoppelt sind, der spezifisch, im Wesentlichen unter Ausschluss anderer Populationen, an vermutete zirkulierende Tumorzellen bindet, und
b. Unterwerfen des Sonde-immunmagnetisches-Partikel-Gemischs einem Hoch-Gradienten-Magnetfeld, um eine abgetrennte Zellfraktion zu erhalten, die an immunmagnetische Partikel gebundenen Tumorzellen angereichert ist.

3. Verfahren nach Anspruch 1, wobei das phänotypische Profil anhand eines Verfahrens bestimmt wird, das aus einer aus Multiparameter-Durchflusszytometrie, Immunfluoreszenzmikroskopie, Laser-Scanning-Zytometrie, Hellfeld-Basis-Bildanalyse, Kapillarvolumetrie, spektraler Bildgebungsanalyse, manueller Zellanalyse, automatisierter Zellanalyse und Kombinationen davon bestehenden Gruppe ausgewählt wird.

4. Verfahren nach Anspruch 3, wobei das phänotypische Profil anhand einer automatisierten Immunfluoreszenz-Zellanalyse bestimmt wird.

5. Verfahren nach Anspruch 4, wobei die Klonsequenz aus künstlichen Bakterienchromosomen-Klonen ausgewählt wird.

6. Verfahren nach Anspruch 1, wobei die hybridisierende vermutete Zielzelle diagnostische, prognostische oder therapeutische Informationen des Patienten liefert.

7. Kit zur Bestimmung von IGF-1R-Gen-Aberrationen in zirkulierenden Tumorzellen aus einer Patientenprobe, umfassend:
a. eine Repeat-freie IGF-1R-Sondenkonfiguration, die in der Lage ist, Chromosomenaberrationen zu detektieren und die aus Klonsequenzen besteht, die mindestens einen Abschnitt des IGF-1R-Locus enthalten,
b. eine Repeat-freie Sonde ohne Kreuzhybridisierung, die für die ql-Region von Chromosom 15 spezifisch ist, und
c. an (a) und (b) gebundene Markierungseinheiten.

8. Kit nach Anspruch 7, wobei die Polynukleotidsondensequenz an den repetitiven Sequenzen durch Verdau mit Duplex-spezifischer Nuklease verarmt worden ist.

9. Kit nach Anspruch 7, wobei die Markierungseinheit ein Fluorophor ist, der durch eine auf Platin basierende koordinative Bindung gebunden ist.

## Revendications

1. Procédé d'évaluation d'aberrations du gène IGF-1R dans des cellules tumorales circulantes dans un échantillon de sang obtenu à partir d'un patient, ledit échantillon comprenant une population de cellules mixtes suspectée de contenir des cellules tumorales circulantes, comprenant :
a. isolement d'une sous-population de cellules épithéliales par enrichissement immunomagnétique ;
b. identification de cellules tumorales circulantes suspectes ; et
c. hybridation desdites cellules tumorales circulantes suspectes avec une configuration de sonde sans répétition d'IFG-1R capable de détecter des aberrations chromosomiques qui est constituée de séquences de clone contenant au moins une partie du locus IGF-1R et une sonde sans répétition sans hybridation croisée spécifique pour la région ql du chromosome 15.

2. Procédé de la revendication 1 dans lequel ledit enrichissement immunomagnétique comprend :
a. le mélange dudit échantillon avec des particules immunomagnétiques colloïdales couplées à un ligand qui se lie spécifiquement à des cellules tumorales circulantes suspectes, à l'exclusion substantielle d'autres populations ; et
b. la soumission du mélange échantillon-particules immunomagnétiques à un champ magnétique à gradient élevé pour produire une fraction de cellules séparées enrichie en cellules tumorales liées aux particules immunomagnétiques.

3. Procédé de la revendication 1 dans lequel ledit profil phénotypique est déterminé par un procédé choisi dans un groupe constitué de la cytométrie en flux multiparamétrique, la microscopie par immunofluorescence, la cytométrie à balayage laser, l'analyse d'image sur fond clair, la volumétrie capillaire, l'analyse par imagerie spectrale, l'analyse de cellules manuelle, l'analyse de cellules automatisée et des combinaisons de celles-ci.

4. Procédé de la revendication 3 dans lequel ledit profil phénotypique est déterminé par analyse de cellules par immunofluorescence automatisée.

5. Procédé de la revendication 4 dans lequel ladite séquence de clone est sélectionnée parmi des clones de chromosome artificiel bactérien.

6. Procédé de la revendication 1 dans lequel ladite cellule cible suspecte s'hybridant fournit des informations diagnostiques, pronostiques ou thérapeutiques dudit patient.

7. Kit de détermination d'aberrations du gène IGF-1R dans des cellules tumorales circulantes d'un échantillon de patient comprenant :
a. une configuration de sonde sans répétition d'IFG-1R capable de détecter des aberrations chromosomiques qui est constituée de séquences de clone contenant au moins une partie du locus IGF-1R ;
b. une sonde sans répétition sans hybridation croisée spécifique pour la région ql du chromosome 15 ; et
c. des fragments de marquage liés auxdites (a) et (b).

8. Kit de la revendication 7 dans lequel ladite séquence de sonde polynucléotidique est dépourvue desdites séquence répétitifs par digestion avec une nucléase spécifique de double hélice.

9. Kit de la revendication 7 dans lequel ledit fragment de marquage est un fluorophore lié par une liaison de coordination à base de platine.
